# EUROPÄISCHE PATENTSCHRIFT

(11) **EP 3 176 152 B1**
(45) Veröffentlichungstag und Bekanntmachung des Hinweises auf die Patenterteilung: **15.05.2019**
(21) Anmeldenummer: 16000734.0
(22) Anmeldetag: 30.03.2016
(51) Int. Cl.: C07C 273/10, B01D 53/04, B01D 53/047, C01B 3/36, C10G 2/00, C01B 32/50, C01B 3/48, C01C 1/04, C10K 1/32, C10K 3/04

(54) **VERFAHREN ZUR ERZEUGUNG VON HARNSTOFF**
METHOD FOR PREPARING UREA
PROCEDE DE FABRICATION D'UREE

(30) Priorität: 01.12.2015 DE 102015015524
(43) Veröffentlichungstag der Anmeldung: 07.06.2017
(73) Patentinhaber: Linde Aktiengesellschaft, 80331 München (DE)
(72) Erfinder: Schwarzhuber, Josef, 85283 Wolnzach (DE); Seliger, Andreas, 80796 München (DE); Voss, Christian, 82538 Geretsried (DE); Wawrzinek, Klemens, 82031 Grünwald (DE); Mabrouk, Rachid, 81477 München (DE); Salazar, Gabriel Duarte, 80336 München (DE); Schürer, Benedikt, 82049 Pullach (DE)

(56) Entgegenhaltungen:
- WO-A1-2015/117738
- CHRISTENSEN ET AL: "Adiabatic prereforming of hydrocarbons - an important step in syngas production", APPLIED CATALYSIS A: GENERAL, ELSEVIER, AMSTERDAM, NL, Bd. 138, Nr. 2, 9. Mai 1996 (1996-05-09), Seiten 285-309, XP022250773, ISSN: 0926-860X, DOI: 10.1016/0926-860X(95)00302-9
- Gaetano Iaquaniello ET AL: "Natural Gas Catalytic Partial Oxidation: A Way to Syngas and Bulk Chemicals Production" In: "Natural Gas - Extraction to End Use", 31 October 2012 (2012-10-31), InTech, XP055247808, ISBN: 978-953-51-0820-7 DOI: 10.5772/48708,

## Beschreibung

Die Erfindung betrifft ein Verfahren zur Erzeugung von Harnstoff gemäß Anspruch 1.

Die Harnstoffsynthese (H₂N-CO-NH₂) benötigt zwei wichtige Reaktanden, nämlich CO₂ und Ammoniak (NH₃).

Die Ammoniaksynthese zur Erzeugung des benötigten Ammoniaks bzw. die Harnstoffsynthese kann dabei auf einer Dampfreformierung basiert sein, die den benötigten Wasserstoff für die Ammoniaksynthese bzw. CO₂ für die Harnstoffsynthese bereitstellt. Hierbei erzeugtes CO₂ wird üblicherweise mittels eines Waschverfahrens entfernt, wobei das CO₂ in der Regel beim Regenerieren des mit CO₂ beladenen Waschmittels erzeugt wird. Hierzu wird das beladene Waschmittel typischer Weise bei einem vergleichsweise niedrigen Druck erhitzt, so dass ein hoher Energiebedarf für die bei der Harnstoffsynthese erforderliche Verdichtung des CO₂ entsteht.

Ammoniak und CO₂ sind die Hauptreaktanden für die Harnstoffsynthese. Ammoniak wird für gewöhnlich mittels luftgespeister ATR-Reformer produziert, wobei erzeugtes H₂ und N₂ gemischt werden, gefolgt von einem Shift-Reaktor und einem Methanisierungsreaktor zum Konvertieren des gesamten CO zu Methan. CO₂ und Methan werden danach von dem H₂-N₂-Gemisch getrennt. Das H₂-N₂-Gemisch wird sodann verdichtet und im Ammoniak-Reaktor konvertiert. In einem alternativen Layout kann Ammoniak erzeugt werden, indem Wasserstoff aus einem Dampfreformer mit Stickstoff aus einer Luftzerlegungseinheit umgesetzt wird. Dieses Layout erfordert zusätzlich zu einer Luftzerlegungseinheit eine konventionelle Wasserstoffanlage. Wasserstoff und Stickstoff werden vor der Einspeisung in den Ammoniakreaktor gemischt und verdichtet. Der Vorteil des zweiten Layouts besteht in dem geringen Inertengehalt des Ammoniaksynthesegases.

Das erzeugte Ammoniak wird dann durch Zufügen von CO₂ zu Harnstoff umgesetzt. Regelmäßig reicht das in beiden Layouts erzeugte CO₂ nicht aus, um das erzeugte Ammoniak vollständig umzusetzen. Daher wird jeweils CO₂ von externen Quellen importiert sofern vorhanden.
laquaniello offenbart in "Natural Gas - Extraction to End Use, Chapter 12: Natural Gas Catalytic Partial Oxidation: A Way to Syngas and Bulk Chemicals Production", ein Verfahren zur Herstellung von Harnstoff aus Erdgas, siehe Figure 9, sowie die Seiten 276 und 277. Das bei der Wassergas-Shift-Reaktion entstehende Kohlendioxid wird in einer separaten Reaktoreinheit aus der Mischung mit Wasserstoff entfernt. Dabei wird das Kohlendioxid in einer Amin-, bzw. Carbonatlösung absorbiert, siehe insbesondere Seite 273.

Der Erfindung liegt vor diesem Hintergrund die Aufgabe zugrunde, ein Verfahren der eingangs genannten Art zu verbessern.

Diese Aufgabe wird durch ein Verfahren mit den Merkmalen des Anspruchs 1 gelöst. Danach ist erfindungsgemäß vorgesehen, dass das Verfahren zum Herstellen von Harnstoff (H₂N-CO-NH₂), die Schritte aufweist:
- Umsetzen eines methanhaltigen sowie vorzugsweise entschwefelten Einsatzgasstroms (insbesondere aufweisend Erdgas oder CH₄) mit Sauerstoff durch partielle Oxidation zu einem Synthesegasstrom aufweisend Wasserstoff und Kohlenstoffmonoxid,
- Umsetzen von Kohlenstoffmonoxid des Synthesegasstroms in einer Wassergas-Shift-Reaktion mit Wasser zu Kohlenstoffdioxid und Wasserstoff,
- Aufteilen des Synthesegasstroms in zumindest einen ersten und einen zweiten Synthesegasteilstrom,
- Abtrennen von Wasserstoff aus dem ersten Synthesegasteilstrom mittels Druckwechseladsorption, und
- Abtrennen von Kohlenstoffdioxid aus dem zweiten Synthesegasteilstrom mittels Temperaturwechseladsorption,
- Umsetzen von aus dem ersten Synthesegasteilstrom abgetrenntem Wasserstoff (H₂) mit Stickstoff (N₂) zu Ammoniak (NH₃), und
- Umsetzen von Ammoniak mit aus dem zweiten Synthesegasteilstrom abgetrenntem Kohlenstoffdioxid (CO₂) zu Harnstoff.

Für die Synthesegaserzeugung wird danach bevorzugt die partielle Oxidation (POX) verwendet. Hierbei kann sowohl eine katalysatorbasierte POX als auch eine POX verwendet werden, die ohne einen Katalysator auskommt.

Der Einsatzgasstrom weist bevorzugt einen oder mehrere der folgenden Komponenten bzw. Kohlenwasserstoffe auf, die im Synthesegaserzeugungsschritt zu dem Synthesegas umgesetzt werden, das H₂ und CO aufweist: Erdgas, CH₄, H₂O, CO₂. Bei der partiellen Oxidation wird der vorzugsweise vorgereinigte, insbesondere entschwefelte (siehe auch unten) Einsatzgasstrom, der z.B. Erdgas bzw. CH₄ oder höhere Kohlenwasserstoffe wie Naphtha, LPG, Öl oder auch Kohle aufweist, insbesondere substöchiometrisch in einem exothermen Prozess umgesetzt. Reaktionsprodukte sind vor allem die das Synthesegas bildenden Stoffe Wasserstoff und Kohlenstoffmonoxid, die gemäß

CₙHₘ + (n+x)/2O₂ ↔ (n-x+y/2)CO + (m-y)/2H₂ + (x-y/2)CO₂ + y/2H₂O

erhalten werden. Bei der partiellen Oxidation kann auch Wasserdampf als Reaktand hinzugefügt werden.

Bei der besagten Wassergas-Shift-Reaktion, der der durch POX erzeugte Synthesegasstrom unterzogen wird, wird gemäß

CO + H₂O <-> CO₂ + H₂

im Synthesegas enthaltenes CO mit Wasser zu Kohlenstoffdioxid und Wasserstoff umgesetzt, was hier besonders vorteilhaft ist, da einerseits Wasserstoff für die Ammoniaksynthese und CO₂ für die Harnstoffsynthese benötigt werden.

Gemäß einer Ausführungsform der Erfindung ist weiterhin vorgesehen, dass das abgetrennte Kohlendioxid bei der Abtrennung mit einem hohen Druck von zumindest 20 bar, bevorzugt zumindest 30bar, am meisten bevorzugt zumindest 50 bar bereitgestellt wird.

Gemäß einer Ausführungsform der Erfindung ist weiterhin vorgesehen, dass das abgetrennte Kohlendioxid für die Umsetzung von Ammoniak zu Harnstoff zumindest stöchiometrisch bereitgestellt wird, so dass der Ammoniak (NH₃) vollständig zu Harnstoff umgesetzt wird.

Gemäß einer Ausführungsform der Erfindung ist weiterhin vorgesehen, dass bei der Temperaturwechseladsorption zum Abtrennen des CO₂ während einer Zykluszeit CO₂ aus dem zweiten Synthesegasteilstrom an einem Adsorber adsorbiert und anschließend desorbiert wird, wobei die Zykluszeit vorzugsweise kleiner ist als 360 min, bevorzugt kleiner als 240 min, am meisten bevorzugt kleiner als 180 min.

Gemäß einer Ausführungsform der Erfindung ist weiterhin vorgesehen, dass zum Abtrennen des Wasserstoffs aus dem ersten Synthesegasteilstrom bei der Druckwechseladsorption im ersten Synthesegasteilstrom enthaltenes CO₂ und CO (sowie insbesondere CH₄) an einem Adsorber bei einem ersten Druck adsorbiert werden, wobei vorzugsweise der Adsorber bei einem zweiten Druck, der niedriger ist als der erste Druck, regeneriert wird, wobei adsorbiertes CO₂ und CO (sowie insbesondere CH₄) desorbiert werden, und wobei der Adsorber zum Entfernen des desorbierten CO₂ und CO (sowie insbesondere CH₄) z.B. mit Wasserstoff unter Erzeugung eines entsprechenden Abgases gespült wird.

Gemäß einer Ausführungsform der Erfindung ist weiterhin vorgesehen, dass das Abgas der Druckwechseladsorption als Brennstoff verwendet wird, wobei vorzugsweise das Abgas zum Heizen des Einsatzgasstromes und/oder zum Erzeugen und/oder Überhitzen von Wasserdampf verbrannt wird. Weiterhin können Abgase zur Erzeugung von Energie verbrannt werden oder ggf. erneut verdichtet und in die POX zurückgeführt werden.

Gemäß einer Ausführungsform der Erfindung ist weiterhin vorgesehen, dass ein bei der Temperaturwechseladsorption erzeugtes Abgas aufweisend H₂ und CO (sowie insbesondere CH₄) ebenfalls einer Druckwechseladsorption unterzogen wird, um zusätzlich Wasserstoff für die Erzeugung des Ammoniaks bereitzustellen, wobei vorzugsweise jenes Abgas aus der Temperaturwechseladsorption zusammen mit dem ersten Synthesegasteilstrom der besagten Druckwechseladsorption unterzogen wird, und/oder dass das Abgas aus der Temperaturwechseladsorption mit dem bei der Druckwechseladsorption anfallenden Abgas (aufweisend CO₂ und CO und insbesondere CH₄) vermischt wird und als Brennstoff verwendet wird.

Gemäß einer Ausführungsform der Erfindung ist weiterhin vorgesehen, dass im (bei der Temperaturwechseladsorption abgetrennten) CO₂ enthaltene Verunreinigungen (z.B. in Form von H₂, CH₄, und/oder CO) in einem Reinigungsschritt, vorzugsweise mittels katalytischer Oxidation, stromauf der Umsetzung des CO₂ mit dem Ammoniak zu dem Harnstoff entfernt werden.

Gemäß einer Ausführungsform der Erfindung ist weiterhin vorgesehen, dass der Synthesegasstrom stromauf und/oder stromab der Wassergas-Shift-Reaktion gekühlt wird, wobei der Synthesegasstrom vorzugsweise mit Wasser unter Erzeugung von Prozessdampf abgekühlt wird.

Gemäß einer Ausführungsform der Erfindung ist weiterhin vorgesehen, dass beim Abkühlen anfallende Wärme zum Regenerieren eines Adsorbers bei der Temperaturwechseladsorption verwendet wird.

Gemäß einer Ausführungsform der Erfindung ist weiterhin vorgesehen, dass der für die POX benötigte Sauerstoff durch kryogene Zerlegung von Luft erzeugt wird, wobei bei jener Zerlegung weiterhin der Stickstoff erzeugt wird, der mit Wasserstoff zu Ammoniak umgesetzt wird.

Gemäß einer Ausführungsform der Erfindung ist weiterhin vorgesehen, dass der Einsatzgasstrom stromauf der partiellen Oxidation durch eine Adsorbereinheit geführt wird, wobei eine oder mehrere noch im Einsatzgasstrom befindliche Schwefelverbindungen in der Adsorbereinheit adsorbiert und dabei aus dem Einsatzgasstrom entfernt werden.

Gemäß einer Ausführungsform der Erfindung ist weiterhin vorgesehen, dass der Synthesegasstrom oder die beiden Synthesegasteilströme stromab der Wassergas-Shift-Reaktion sowie stromauf der Druckwechseladsorption sowie Temperaturwechseladsorption getrocknet werden.

Gemäß einem weiteren Aspekt der Erfindung wird eine Anlage zur Herstellung von Harnstoff mit den Merkmalen des Anspruchs 15 vorgeschlagen.

Danach weist die Anlage zum Herstellen von Harnstoff, auf:
- einen POX-Reaktor der zum Umsetzen eines methanhaltigen sowie vorzugsweise entschwefelten Einsatzgasstroms mit Sauerstoff durch partielle Oxidation zu einem Synthesegasstrom aufweisend Wasserstoff und Kohlenstoffmonoxid konfiguriert ist,
- einen Wassergas-Shift-Reaktor stromab des POX-Reaktors, der zum Umsetzen von Kohlenstoffmonoxid des Synthesegasstroms in einer Wassergas-Shift-Reaktion mit Wasser zu Kohlenstoffdioxid und Wasserstoff konfiguriert ist, wobei die Anlage weiterhin dazu konfiguriert ist, den aus dem Wassergas-Shift-Reaktor kommenden Synthesegasstrom in zumindest einen ersten und einen zweiten Synthesegasteilstrom aufzuteilen,
- eine Druckwechseladsorptionseinheit, die dazu konfiguriert ist, den ersten Synthesegasteilstrom einer Druckwechseladsorption zu unterziehen, wobei Wasserstoff vom ersten Synthesegasteilstrom abgetrennt wird, und
- eine Temperaturwechseladsorptionseinheit, die dazu konfiguriert ist, den zweiten Synthesegasteilstrom einer Temperaturwechseladsorption zu unterziehen, wobei Kohlenstoffdioxid vom zweiten Synthesegasteilstrom abgetrennt wird,
- einen Ammoniak-Reaktor, der zum Umsetzen von aus dem ersten Synthesegasteilstrom abgetrenntem Wasserstoff mit Stickstoff zu Ammoniak konfiguriert ist, und
- einem Harnstoff-Reaktor, der zum Umsetzen von Ammoniak mit aus dem zweiten Synthesegasteilstrom abgetrenntem Kohlenstoffdioxid zu Harnstoff konfiguriert ist.

Die erfindungsgemäße Anlage wird im Übrigen in weiteren Ausführungsformen durch die entsprechenden Ausführungsformen des erfindungsgemäßen Verfahrens charakterisiert. Diesbezüglich ist die Anlage vorzugsweise jeweils dazu konfiguriert, die entsprechenden Verfahrensschritte der jeweiligen Ausführungsform des erfindungsgemäßen Verfahrens durchzuführen.

Weitere Merkmale und Vorteile der Erfindung werden nachfolgend bei der Figurenbeschreibung von Ausführungsbeispielen der Erfindung anhand der Figuren erläutert. Es zeigen:
- Fig. 1: eine schematische Darstellung eines erfindungsgemäßen Verfahrens zur Herstellung von Harnstoff; und
- Fig. 2: eine schematische Darstellung der Abtrennung von CO₂ und H₂ aus einem beim erfindungsgemäßen Verfahrens erzeugten Synthesegas.

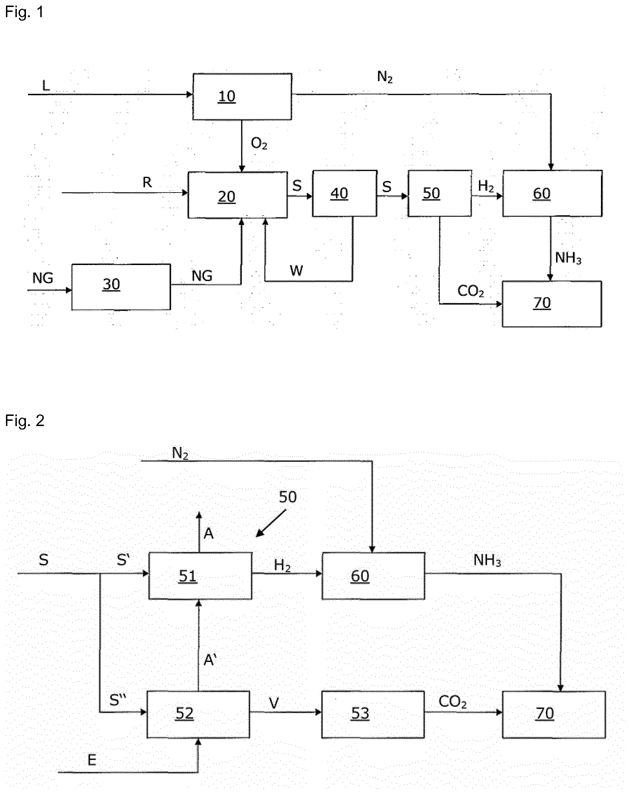

Figur 1 zeigt eine schematische Darstellung einer Anlage bzw. eines Verfahrens zum Herstellen von Harnstoff.

Hierbei wird ein z.B. CH₄ aufweisender Einsatzgasstrom NG (z.B. in Form von Erdgas) vor einer Umsetzung zu Synthesegas (aufweisend H₂ und CO) S durch partielle Oxidation 20 einer Entschwefelung 30 unterzogen und sodann mittels partieller Oxidation 20 in Gegenwart von Sauerstoff sowie insbesondere Wasserdampf W zu einem Synthesegasstrom S umgesetzt, der H₂ und CO aufweist sowie weiterhin insbesondere CH₄, H₂O und CO₂.

Der Synthesegasstrom S wird hiernach einer Wassergas-Shift-Reaktion 40 unterzogen (siehe oben) und mit Wasser abgekühlt, wobei der besagte Wasserdampf W erzeugt werden kann. Grundsätzlich kann beim Abkühlen des Synthesegases S anfallende Wärme auch zur Regeneration von Adsorbern in der weiter unten beschriebenen Temperaturwechseladsorption 52 verwendet werden (vgl. Fig. 2).

Der Synthesegasstrom S wird weiterhin getrocknet, wobei anschließend Wasserstoff und Kohlenstoffdioxid des Synthesegasstromes S getrennt werden (50), wobei der Wasserstoff mit Stickstoff zu Ammoniak umgesetzt wird (60), und wobei das Kohlenstoffdioxid mit dem erzeugten Ammoniak letztlich zu Harnstoff umgesetzt wird.

Der Sauerstoff für die POX 20 wird durch kryogene Zerlegung 10 von Luft L erzeugt, wobei auch jener Stickstoff gewonnen wird, der für die Ammoniaksynthese 60 benötigt wird

Gemäß Figur 2 erfolgt die Abtrennung 50 von Wasserstoff und Kohlenstoffdioxid dergestalt, dass der geshiftete Synthesegasstrom S in einen ersten und einen zweiten Synthesegasteilstrom S', S" unterteilt wird, wobei der erste Synthesegasteilstrom S' einer Druckwechseladsorption 51 unterzogen wird, wobei Wasserstoff vom ersten Synthesegasteilstrom S' abgetrennt wird, und wobei der zweite Synthesegasteilstrom S" einer Temperaturwechseladsorption 52 (siehe oben) unterzogen wird, die vorzugsweise zumindest teilweise indirekt, z.B. über ein Wärmeträgermedium, das nicht in direktem Kontakt mit dem Adsorbens ist, geheizt und/oder gekühlt wird, wobei Kohlenstoffdioxid vom zweiten Synthesegasteilstrom S" abgetrennt wird. Die Zykluszeiten einer solchen Temperaturwechseladsorption sind meist kurz und liegen im Bereich 2 bis 6 Stunden. Der abgetrennte Wasserstoff wird dann zusammen mit dem Stickstoff zu Ammoniak 60 umgesetzt, das wiederum mit dem abgetrennten CO₂ zu Harnstoff 70 umgesetzt wird. Vorzugsweise wird das aus der Temperaturwechseladsorption 52 kommende CO₂ V stromauf der Harnstoffsynthese 70 noch weiter aufgereinigt 53, insbesondere um darin enthaltene Verunreinigungen, wie z.B. H₂, CH₄ und CO, Methanol, zu entfernen.

Bei der Druckwechseladsorption 51 zum Abtrennen des Wasserstoff aus dem ersten Synthesegasteilstrom S' wird im ersten Synthesegasteilstrom enthaltenes CO₂ und CO sowie ggf. weitere Komponenten (wie z.B. CH₄) an einem Adsorber bei einem ersten Druck adsorbiert, wobei vorzugsweise der Adsorber bei einem zweiten Druck, der niedriger ist als der erste Druck, regeneriert wird, wobei die adsorbierten Komponenten desorbiert werden, und wobei der Adsorber zum Entfernen der desorbierten Komponenten unter Erzeugung eines Abgases A gespült wird. Vorzugsweise werden mehrere, insbesondere zwei oder vier Adsorber bei der Druckwechseladsorption 51 verwendet, damit möglichst immer ein Adsorber im Adsorptionsmodus betrieben werden kann, so dass quasi kontinuierlich Wasserstoff abgegeben werden kann.

Das Abgas A aus der Druckwechseladsorption 51 kann z.B. als Brennstoff verwendet werden, wobei z.B. das Abgas A zum Heizen des Einsatzgasstromes NG und/oder zum Erzeugen und/oder Überhitzen von Wasserdampf verbrannt werden kann.

Bei der Temperaturwechseladsorption 52 wird CO₂ bei einer niedrigen ersten Temperatur an einem Adsorber adsorbiert und bei einer höheren zweiten Temperatur desorbiert, wofür die notwendige Energie E bereitgestellt wird. Das beim Adsorbieren von CO₂ anfallende Restgas bzw. Abgas A', dass H₂ und CO aufweist, kann zusammen mit dem ersten Synthesegasteilstrom S' in die Druckwechseladsorption 51 gefahren werden oder kann mit dem Abgas A aus der Druckwechseladsorption 51 vermischt werden und mit diesem zusammen als Brennstoff verwendet werden.

Aufgrund der erfindungsgemäßen Abtrennung von CO₂ liegt dieses nach dem Abtrennen in vorteilhafter Weise mit einem hohem Druck von vorzugsweise zumindest 20 bar vor, so dass entsprechend Energie für die ansonsten notwendige Verdichtung des CO₂ zum Zwecke der Harnstoffsynthese eingespart werden kann. Dies liegt vornehmlich daran, dass bei der Temperaturwechseladsorption mittels Erhitzen des Adsorbens regeneriert wird, so dass der im Vergleich bei einer Regeneration in der Druckwechseladsorption auftretende Druckverlust vermeidbar ist.

Weiterhin muss in Gegenwart der CO₂-Aufreinigung 53 per katalytischer Oxidation mit Vorteil das aus der Temperaturwechseladsorption kommende CO₂ nicht gekühlt werden, da dieses für die katalytische Oxidation eine entsprechend erhöhte Temperatur aufweisen muss.

Die Verwendung einer entsprechend ausgelegten katalytischen Oxidation kann die bei der Desorption entstehenden Zusammensetzungsschwankungen ausgleichen und so eine möglichst gleichmäßige CO₂-Qualität gewährleisten. Es kann beispielsweise die Regelung derart angepasst werden, dass der Sauerstoffbedarf der katalytischen Oxidation berücksichtigt wird, und so immer eine möglichst konstante Sauerstoffkonzentration im CO₂, beispielsweise unter 0,7 Vol.-%, insbesondere unter 0,6 Vol.-% oder insbesondere < 0.35 Vol.-%, eingehalten wird. Diese Regelmöglichkeit ist vorteilhaft für die Stabilität und die Energieeffizienz der anschließenden Harnstoff-Anlage. Für die Regelung des O₂-Gehalts im CO₂ kann die Desorption der brennbaren Komponenten aufgrund der Heizung vorausberechnet werden. Dementsprechend kann dann die Luftmenge eingestellt werden. Es besteht auch die Möglichkeit z.B. zusätzlich den O₂-Gehalt im CO₂ zu messen und zu regeln.

Im Ergebnis ermöglicht die Erfindung die Integration bekannter Technologien, wie z.B. POX, ASU (kryogene Luftzerlegung), Druckwechseladsorption und Temperaturwechseladsorption in ein Anlagen- bzw. Verfahrenskonzept, das ausreichend CO₂ für die Harnstoffsynthese bereitstellen kann, so dass eine vollständige Umsetzung des erzeugten Ammoniaks möglich ist, wobei das benötigte CO₂ auf einem hohen Druckniveau bereitgestellt wird, so dass auf eine kostenintensive zusätzliche Verdichtung verzichtet werden kann.

**Bezugszeichenliste**

| | |
|---|---|
| 10 | Luftzerlegung |
| 20 | POX |
| 30 | Entschwefelung |
| 40 | Wassergas-Shift-Reaktion und Kühlung des Synthesegases |
| 50 | Abtrennung H₂ und CO₂ |
| 51 | Druckwechseladsorption |
| 52 | Temperaturwechseladsorption |
| 53 | CO₂-Reinigung |
| 60 | Ammoniaksynthese |
| 70 | Harnstoffsynthese |
| A, A' | Abgas |
| E | Energie zum Heizen |
| L | Luft |
| NG | Einsatzgas |
| S | Synthesegas |
| S' | Erster Synthesegasteilstrom |
| S" | Zweiter Synthesegasteilstrom |
| R | Geshiftetes Synthesegas Rücklauf |
| V | CO2 mit Verunreinigungen stromab Temperaturwechseladsorption |
| W | Wasserdampf |

## Patentansprüche

1. Verfahren zum Herstellen von Harnstoff, aufweisend die Schritte:
- Umsetzen eines methanhaltigen sowie vorzugsweise entschwefelten Einsatzgasstroms (NG) mit Sauerstoff durch partielle Oxidation (20) zu einem Synthesegasstrom (S) aufweisend Wasserstoff und Kohlenstoffmonoxid,
- Umsetzen von Kohlenstoffmonoxid des Synthesegasstroms (S) in einer Wassergas-Shift-Reaktion (40) mit Wasser zu Kohlenstoffdioxid und Wasserstoff,
- Aufteilen des Synthesegasstroms (S) in zumindest einen ersten und einen zweiten Synthesegasteilstrom (S', S"),
- wobei der erste Synthesegasteilstrom (S') einer Druckwechseladsorption (51) unterzogen wird, wobei Wasserstoff vom ersten Synthesegasteilstrom (S') abgetrennt wird, und
- wobei der zweite Synthesegasteilstrom (S") einer Temperaturwechseladsorption (52) unterzogen wird, wobei Kohlenstoffdioxid vom zweiten Synthesegasteilstrom (S") abgetrennt wird
- Umsetzen (60) von aus dem ersten Synthesegasteilstrom (S') abgetrenntem Wasserstoff mit Stickstoff zu Ammoniak, und
- Umsetzen (70) von Ammoniak mit aus dem zweiten Synthesegasteilstrom (S") abgetrenntem Kohlenstoffdioxid zu Harnstoff.

2. Verfahren nach Anspruch 1, **dadurch gekennzeichnet, dass** das abgetrennte Kohlenstoffdioxid bei der Abtrennung (52) mit einem hohem Druck von zumindest 10 bar, bevorzugt zumindest 20 bar, am meisten bevorzugt zumindest 50 bar bereitgestellt wird.

3. Verfahren nach Anspruch 1 oder 2, **dadurch gekennzeichnet, dass** das abgetrennte Kohlenstoffdioxid für die Umsetzung (70) von Ammoniak zu Harnstoff zumindest stöchiometrisch bereitgestellt wird, so dass der Ammoniak vollständig zu Harnstoff umgesetzt wird.

4. Verfahren nach einem der vorhergehenden Ansprüche, **dadurch gekennzeichnet, dass** bei der Temperaturwechseladsorption (52) Kohlenstoffdioxid aus dem zweiten Synthesegasteilstrom (S") adsorbiert und anschließend desorbiert wird, wobei ein bei der Temperaturwechseladsorption verwendetes Adsorbens vorzugsweise zumindest teilweise indirekt, insbesondere über ein Wärmeträgermedium, das nicht in direktem Kontakt mit dem Adsorbens steht, geheizt und / oder gekühlt wird.

5. Verfahren nach einem der vorhergehenden Ansprüche, **dadurch gekennzeichnet, dass** bei der Temperaturwechseladsorption (52) zum Abtrennen des Kohlenstoffdioxids während einer Zykluszeit Kohlenstoffdioxid aus dem zweiten Synthesegasteilstrom (S") in einer Adsorberstation adsorbiert und anschließend desorbiert wird, wobei die Zykluszeit vorzugsweise kleiner ist als 360 min, bevorzugt kleiner als 240 min, am meisten bevorzugt kleiner als 180 min.

6. Verfahren nach einem der vorhergehenden Ansprüche, **dadurch gekennzeichnet, dass** zum Abtrennen des Wasserstoff aus dem ersten Synthesegasteilstrom (S') bei der Druckwechseladsorption (51) im ersten Synthesegasteilstrom (S') enthaltenes CO₂ und CO an einem Adsorber (51) bei einem ersten Druck adsorbiert werden, wobei vorzugsweise der Adsorber bei einem zweiten Druck, der niedriger ist als der erste Druck, regeneriert wird, wobei adsorbiertes CO₂ und CO desorbiert werden, und wobei der Adsorber zum Entfernen des desorbierten CO₂ und CO unter Erzeugung eines Abgases (A) gespült wird.

7. Verfahren nach Anspruch 6, **dadurch gekennzeichnet, dass** das Abgas (A) der Druckwechseladsorption (51) als Brennstoff verwendet wird, wobei vorzugsweise das Abgas (A) zum Heizen des Einsatzgasstromes (NG) und/oder zum Erzeugen und/oder Überhitzen von Wasserdampf verbrannt wird.

8. Verfahren nach einem der vorhergehenden Ansprüche, **dadurch gekennzeichnet, dass** ein bei der Temperaturwechseladsorption (52) erzeugtes Abgas (A') aufweisend H₂ und CO ebenfalls einer Druckwechseladsorption unterzogen wird, um zusätzlich Wasserstoff für die Erzeugung des Ammoniaks bereitzustellen, wobei vorzugsweise jenes Abgas (A') aus der Temperaturwechseladsorption (52) zusammen mit dem ersten Synthesegasteilstrom (S') der besagten Druckwechseladsorption (51) unterzogen wird, und/oder dass das Abgas (A') aus der Temperaturwechseladsorption (52) mit dem bei der Druckwechseladsorption (51) anfallenden Abgas (A) vermischt wird und als Brennstoff verwendet wird.

9. Verfahren nach einem der vorhergehenden Ansprüche, **dadurch gekennzeichnet, dass** das bei der Temperaturwechseladsorption (52) abgetrenntes CO₂ (V) Verunreinigungen in Form von zumindest einem der folgenden Stoffe aufweist: H₂, CH₄, CO; wobei die Verunreinigungen in einem Reinigungsschritt (53), vorzugsweise mittels katalytischer Oxidation, stromauf der Umsetzung des CO₂ mit dem Ammoniak zu dem Harnstoff (70) entfernt werden.

10. Verfahren nach einem der vorhergehenden Ansprüche, **dadurch gekennzeichnet, dass** der Synthesegasstrom (S) stromauf und/oder stromab der Wassergas-Shift-Reaktion gekühlt wird (40), wobei der Synthesegasstrom (S) vorzugsweise mit Wasser unter Erzeugung von Prozessdampf (W) abgekühlt wird.

11. Verfahren nach Anspruch 10, **dadurch gekennzeichnet, dass** beim Abkühlen anfallende Wärme zum Regenerieren eines Adsorbers (52) bei der Temperaturwechseladsorption (52) verwendet wird.

12. Verfahren nach einem der vorhergehenden Ansprüche, **dadurch gekennzeichnet, dass** der Sauerstoff durch kryogene Zerlegung (10) von Luft (L) erzeugt wird, wobei bei jener Zerlegung (10) weiterhin der Stickstoff erzeugt wird, der mit dem Wasserstoff zu dem Ammoniak umgesetzt wird.

13. Verfahren nach einem der vorhergehenden Ansprüche, **dadurch gekennzeichnet, dass** der Einsatzgasstrom (NG) stromauf der partiellen Oxidation (20) durch eine Adsorbereinheit (30) geführt wird, wobei eine oder mehrere noch im Einsatzgasstrom (NG) befindliche Schwefelverbindungen in der Adsorbereinheit (30) adsorbiert und dabei aus dem Einsatzgasstrom (NG) entfernt werden.

14. Verfahren nach einem der vorhergehenden Ansprüche, **dadurch gekennzeichnet, dass** der Synthesegasstrom (S) oder die beiden Synthesegasteilströme (S', S") stromab der Wassergas-Shift-Reaktion (40) sowie stromauf der Druckwechseladsorption (51) sowie Temperaturwechseladsorption (52) getrocknet werden.

15. Anlage zum Herstellen von Harnstoff, aufweisend
- einen POX-Reaktor (20) zum Umsetzen eines methanhaltigen sowie vorzugsweise entschwefelten Einsatzgasstroms (NG) mit Sauerstoff durch partielle Oxidation (20) zu einem Synthesegasstrom (S) aufweisend Wasserstoff und Kohlenstoffmonoxid,
- einen Wassergas-Shift-Reaktor (40) stromab des POX-Reaktors (20) zum Umsetzen von Kohlenstoffmonoxid des Synthesegasstroms (S) in einer Wassergas-Shift-Reaktion (40) mit Wasser zu Kohlenstoffdioxid und Wasserstoff, wobei die Anlage dazu konfiguriert ist, den aus dem Wassergas-Shift-Reaktor (40) kommenden Synthesegasstrom (S) in zumindest einen ersten und einen zweiten Synthesegasteilstrom (S', S") aufzuteilen,
- eine Druckwechseladsorptionseinheit (51), die dazu konfiguriert ist, den ersten Synthesegasteilstrom (S') einer Druckwechseladsorption (51) zu unterziehen, wobei Wasserstoff vom ersten Synthesegasteilstrom (S') abgetrennt wird, und
- eine Temperaturwechseladsorptionseinheit (52), die dazu konfiguriert ist, den zweiten Synthesegasteilstrom (S") einer Temperaturwechseladsorption (52) zu unterziehen, wobei Kohlenstoffdioxid vom zweiten Synthesegasteilstrom (S") abgetrennt wird,
- einen Ammoniak-Reaktor (60) zum Umsetzen von aus dem ersten Synthesegasteilstrom (S') abgetrenntem Wasserstoff mit Stickstoff zu Ammoniak, und
- einen Harnstoff-Reaktor (70) zum Umsetzen von Ammoniak mit aus dem zweiten Synthesegasteilstrom (S") abgetrenntem Kohlenstoffdioxid zu Harnstoff.

## Claims

1. Method for producing urea, comprising the steps:
- reacting a methane-containing and also, preferably desulphurized, feed gas stream (NG) with oxygen by partial oxidation (20) to form a synthesis gas stream (S) comprising hydrogen and carbon monoxide,
- reacting carbon monoxide of the synthesis gas stream (S) in a water gas-shift reaction (40) with water to form carbon dioxide and hydrogen,
- dividing the synthesis gas stream (S) into at least one first and one second synthesis gas substream (S', S"),
- wherein the first synthesis gas substream (S') is subjected to a pressure-swing adsorption (51), wherein hydrogen is separated off from the first synthesis gas substream (S'), and
- wherein the second synthesis gas substream (S") is subjected to a temperature-swing adsorption (52), wherein carbon dioxide is separated off from the second synthesis gas substream (S")
- reacting (60) hydrogen separated off from the first synthesis gas substream (S') with nitrogen to form ammonia, and
- reacting (70) ammonia with carbon dioxide separated off from the second synthesis gas substream (S") to form urea.

2. Method according to Claim 1, **characterized in that** the carbon dioxide that is separated off in the separation (52) is provided at a high pressure of at least 10 bar, preferably at least 20 bar, most preferably at least 50 bar.

3. Method according to Claim 1 or 2, **characterized in that** the carbon dioxide that is separated off is provided at least stoichiometrically for the reaction (70) of ammonia to form urea, in such a manner that the ammonia is completely reacted to form urea.

4. Method according to any one of the preceding claims, **characterized in that**, during the temperature-swing adsorption (52) carbon dioxide from the second synthesis gas substream (S") is adsorbed and then desorbed, wherein an adsorbent used in the temperature-swing adsorption is heated and/or cooled, preferably at least in part indirectly, in particular via a heat-carrier medium that is not in direct contact with the adsorbent.

5. Method according to one of the preceding claims, **characterized in that**, in the temperature-swing adsorption (52) for separating off the carbon dioxide, during one cycle time, carbon dioxide is adsorbed from the second synthesis gas substream (S") in an adsorber station and is then desorbed, wherein the cycle time is preferably less than 360 min, preferably less than 240 min, most preferably less than 180 min.

6. Method according to one of the preceding claims, **characterized in that**, for separating off the hydrogen from the first synthesis gas substream (S'), CO₂ and CO present in the pressure-swing adsorption (51) in the first synthesis gas substream (S') are adsorbed on an adsorber (51) at a first pressure, wherein, preferably, the adsorber is regenerated at a second pressure that is lower than the first pressure, wherein adsorbed CO₂ and CO are desorbed, and wherein the adsorber, for removing the desorbed CO₂ and CO, is purged with production of an off-gas (A).

7. Method according to Claim 6, **characterized in that** the off-gas (A) of the pressure-swing adsorption (51) is used as fuel, wherein, preferably the off-gas (A) is burnt for heating the feed gas stream (NG) and/or for producing and/or superheating steam.

8. Method according to one of the preceding claims, **characterized in that** an off-gas (A') produced in the temperature-swing adsorption (52) and comprising H₂ and CO is likewise subjected to a pressure-swing adsorption in order additionally to provide hydrogen for manufacture of the ammonia, wherein, preferably, the off-gas (A') from the temperature-swing adsorption (52) is subjected to said pressure-swing adsorption (51) together with the first synthesis gas substream (S'), and/or **in that** the off-gas (A') from the temperature-swing adsorption (52) is mixed with the off-gas (A) arising in the pressure-swing adsorption (51) and used as fuel.

9. Method according to one of the preceding claims, **characterized in that** the CO₂ that is separated off in the temperature-swing adsorption (52) comprises impurities (V) in the form of at least one of the following materials: H₂, CH₄, CO; wherein the impurities are removed in a purification step (53), preferably by means of catalytic oxidation, upstream of the reaction of the CO₂ with the ammonia to form urea (70).

10. Method according to one of the preceding claims, **characterized in that** the synthesis gas stream (S) is cooled (40) upstream and/or downstream of the water gas-shift reaction, wherein the synthesis gas stream (S) is preferably cooled with water, with manufacture of process steam (W).

11. Method according to Claim 10, **characterized in that** heat arising during the cooling is used for regenerating an adsorber (52) in the temperature-swing adsorption (52).

12. Method according to one of the preceding claims, **characterized in that** the oxygen is manufactured by cryogenic separation (10) of air (L), wherein during the separation (10) nitrogen is further manufactured which is reacted with the hydrogen to form ammonia.

13. Method according to one of the preceding claims, **characterized in that** the feed gas stream (NG) is conducted upstream of the partial oxidation (20) through an adsorber unit (30), wherein one or more sulphur compounds that are still present in the feed gas stream (NG) are adsorbed in the adsorber unit (30) and in this case removed from the feed gas stream (NG).

14. Method according to one of the preceding claims, **characterized in that** the synthesis gas stream (S) or the two synthesis gas substreams (S', S") are dried downstream of the water gas-shift reaction (40) and also upstream of the pressure-swing adsorption (51) and also temperature-swing adsorption (52).

15. Plant for producing urea, comprising
- a POX reactor (20) for reacting a methane-containing and also, preferably desulphurized, feed gas stream (NG) with oxygen by partial oxidation (20) to form a synthesis gas stream (S) comprising hydrogen and carbon monoxide,
- a water gas-shift reactor (40) downstream of the POX reactor (20) for reacting carbon monoxide of the synthesis gas stream (S) in a water gas-shift reaction (40) with water to form carbon dioxide and hydrogen, wherein the plant is configured to divide the synthesis gas stream (S) arriving from the water gas-shift reactor (40) into at least one first and one second synthesis gas substream (S', S"),
- a pressure-swing adsorption unit (51) which is configured to subject the first synthesis gas substream (S') to a pressure-swing adsorption (51), wherein hydrogen is separated off from the first synthesis gas substream (S'), and
- a temperature-swing adsorption unit (52), which is configured to subject the second synthesis gas substream (S") to a temperature-swing adsorption (52), wherein carbon dioxide is separated off from the second synthesis gas substream (S"),
- an ammonia reactor (60) for reacting hydrogen separated off from the first synthesis gas substream (S') with nitrogen to form ammonia, and
- a urea reactor (70) for reacting ammonia with carbon dioxide separated off from the second synthesis gas substream (S") to form urea.

## Revendications

1. Procédé de fabrication d'urée, comprenant les étapes suivantes :
- la mise en réaction d'un courant de gaz de charge (NG) contenant du méthane et de préférence désulfuré avec de l'oxygène par oxydation partielle (20) pour former un courant de gaz de synthèse (S) comprenant de l'hydrogène et du monoxyde de carbone,
- la mise en réaction du monoxyde de carbone du courant de gaz de synthèse (S) par une réaction du gaz à l'eau (40) avec de l'eau pour former du dioxyde de carbone et de l'hydrogène,
- la division du courant de gaz de synthèse (S) en au moins un premier et un deuxième courant partiel de gaz de synthèse (S', S"),
- le premier courant partiel de gaz de synthèse (S') étant soumis à une adsorption modulée en pression (51), de l'hydrogène étant séparé du premier courant partiel de gaz de synthèse (S'), et
- le deuxième courant partiel de gaz de synthèse (S") étant soumis à une adsorption modulée en température (52), du dioxyde de carbone étant séparé du deuxième courant partiel de gaz de synthèse (S"),
- la mise en réaction (60) de l'hydrogène séparé du premier courant partiel de gaz de synthèse (S') avec de l'azote pour former de l'ammoniac, et
- la mise en réaction (70) d'ammoniac avec le dioxyde de carbone séparé du deuxième courant partiel de gaz de synthèse (S") pour former de l'urée.

2. Procédé selon la revendication 1, **caractérisé en ce que** le dioxyde de carbone séparé est préparé lors de la séparation (52) avec une pression élevée d'au moins 10 bar, de préférence d'au moins 20 bar, de manière préférée entre toutes d'au moins 50 bar.

3. Procédé selon la revendication 1 ou 2, **caractérisé en ce que** le dioxyde de carbone séparé est préparé en quantité au moins stoechiométrique pour la transformation (70) de l'ammoniac en urée, de telle sorte que l'ammoniac soit entièrement transformé en urée.

4. Procédé selon l'une quelconque des revendications précédentes, **caractérisé en ce que**, lors de l'adsorption modulée en température (52), du dioxyde de carbone est adsorbé à partir du deuxième courant partiel de gaz de synthèse (S"), puis désorbé, un adsorbant utilisé lors de l'adsorption modulée en température étant chauffé et/ou refroidi de préférence au moins partiellement de manière indirecte, notamment par un milieu caloporteur qui n'est pas en contact direct avec l'adsorbant.

5. Procédé selon l'une quelconque des revendications précédentes, **caractérisé en ce que**, lors de l'adsorption modulée en température (52), pour la séparation du dioxyde de carbone, pendant un temps de cycle, du dioxyde de carbone est adsorbé à partir du deuxième courant partiel de gaz de synthèse (S") dans une station d'adsorbeur, puis désorbé, le temps de cycle étant de préférence inférieur à 360 minutes, de préférence inférieur à 240 minutes, de manière préférée entre toutes inférieur à 180 minutes.

6. Procédé selon l'une quelconque des revendications précédentes, **caractérisé en ce que**, pour la séparation de l'hydrogène du premier courant partiel de gaz de synthèse (S') lors de l'adsorption modulée en pression (51), le CO₂ et le CO contenus dans le premier courant partiel de gaz de synthèse (S') sont adsorbés sur un adsorbeur (51) à une première pression, l'adsorbeur étant de préférence régénéré à une deuxième pression, qui est inférieure à la première pression, le CO₂ et le CO adsorbés étant désorbés, et l'adsorbeur étant rincé pour l'élimination du CO₂ et du CO désorbés avec formation d'un gaz d'échappement (A).

7. Procédé selon la revendication 6, **caractérisé en ce que** le gaz d'échappement (A) de l'adsorption modulée en pression (51) est utilisé en tant que combustible, le gaz d'échappement (A) étant de préférence brûlé pour le chauffage du courant de gaz de charge (NG) et/ou pour la formation et/ou le surchauffage de vapeur d'eau.

8. Procédé selon l'une quelconque des revendications précédentes, **caractérisé en ce qu'**un gaz d'échappement (A') formé lors de l'adsorption modulée en température (52), comprenant de l'H₂ et du CO, est également soumis à une adsorption modulée en pression, afin de mettre à disposition davantage d'hydrogène pour la formation de l'ammoniac, le gaz d'échappement (A') issu de l'adsorption modulée en température (52) étant de préférence soumis à ladite adsorption modulée en pression (51) conjointement avec le premier courant partiel de gaz de synthèse (S'), et/ou **en ce que** le gaz d'échappement (A') issu de l'adsorption modulée en température (52) est mélangé avec le gaz d'échappement (A) formé lors de l'adsorption modulée en pression (51) et utilisé en tant que combustible.

9. Procédé selon l'une quelconque des revendications précédentes, **caractérisé en ce que** le CO₂ (V) séparé lors de l'adsorption modulée en température (52) comprend des impuretés sous la forme d'au moins une des substances suivantes : H₂, CH₄, CO ; les impuretés étant éliminées lors d'une étape de purification (53), de préférence par oxydation catalytique, en amont de la mise en réaction du CO₂ avec l'ammoniac pour former l'urée (70).

10. Procédé selon l'une quelconque des revendications précédentes, **caractérisé en ce que** le courant de gaz de synthèse (S) est refroidi (40) en amont et/ou en aval de la réaction du gaz à l'eau, le courant de gaz de synthèse (S) étant de préférence refroidi avec de l'eau avec formation de vapeur de procédé (W).

11. Procédé selon la revendication 10, **caractérisé en ce que** la chaleur formée lors du refroidissement est utilisée pour la régénération d'un adsorbeur (52) lors de l'adsorption modulée en température (52).

12. Procédé selon l'une quelconque des revendications précédentes, **caractérisé en ce que** l'oxygène est formé par décomposition cryogénique (10) d'air (L), l'azote qui est mis en réaction avec l'hydrogène pour former l'ammoniac étant en outre formé lors de la décomposition (10) .

13. Procédé selon l'une quelconque des revendications précédentes, **caractérisé en ce que** le courant de gaz de charge (NG) est passé dans une unité d'adsorbeur (30) en amont de l'oxydation partielle (20), un ou plusieurs composés de soufre encore présents dans le courant de gaz de charge (NG) étant adsorbés dans l'unité d'adsorbeur (30) et ainsi éliminés du courant de gaz de charge (NG).

14. Procédé selon l'une quelconque des revendications précédentes, **caractérisé en ce que** le courant de gaz de synthèse (S) ou les deux courants partiels de gaz de synthèse (S', S") sont séchés en aval de la réaction du gaz à l'eau (40), ainsi qu'en amont de l'adsorption modulée en pression (51) et de l'adsorption modulée en température (52).

15. Unité pour la fabrication d'urée, comprenant :
- un réacteur POX (20) pour la mise en réaction d'un courant de gaz de charge (NG) contenant du méthane et de préférence désulfuré avec de l'oxygène par oxydation partielle (20) pour former un courant de gaz de synthèse (S) comprenant de l'hydrogène et du monoxyde de carbone,
- un réacteur de réaction du gaz à l'eau (40) en aval du réacteur POX (20) pour la mise en réaction de monoxyde de carbone du courant de gaz de synthèse (S) par une réaction du gaz à l'eau (40) avec de l'eau pour former du dioxyde de carbone et de l'hydrogène, l'unité étant configurée de telle sorte que le courant de gaz de synthèse (S) sortant du réacteur de réaction du gaz à l'eau (40) soit divisé en au moins un premier et un deuxième courant partiel de gaz de synthèse (S', S"),
- une unité d'adsorption modulée en pression (51), qui est configurée pour soumettre le premier courant partiel de gaz de synthèse (S') à une adsorption modulée en pression (51), de l'hydrogène étant séparé du premier courant partiel de gaz de synthèse (S'), et
- une unité d'adsorption modulée en température (52), qui est configurée pour soumettre le deuxième courant partiel de gaz de synthèse (S") à une adsorption modulée en température (52), du dioxyde de carbone étant séparé du deuxième courant partiel de gaz de synthèse (S"),
- un réacteur de formation d'ammoniac (60) pour la mise en réaction de l'hydrogène séparé du premier courant partiel de gaz de synthèse (S') avec de l'azote pour former de l'ammoniac, et
- un réacteur de formation d'urée (70) pour la mise en réaction d'ammoniac avec le dioxyde de carbone séparé du deuxième courant partiel de gaz de synthèse (S") pour former de l'urée.
